# EUROPÄISCHE PATENTSCHRIFT

(11) **EP 1 691 701 B1**
(45) Veröffentlichungstag und Bekanntmachung des Hinweises auf die Patenterteilung: **13.06.2012**
(21) Anmeldenummer: 03772466.3
(22) Anmeldetag: 18.11.2003
(51) Int. Cl.: A61B 17/80

(54) **Osteosyntheseplatten-Set**
Set of osteosynthesis plates
Assemblage de plaques d'ostéosynthèse

(43) Veröffentlichungstag der Anmeldung: 23.08.2006
(73) Patentinhaber: Synthes GmbH, 4436 Oberdorf (CH)
(72) Erfinder: KOHUT, Georges, CH-3095 Spiegel bei Bern (CH); INAUEN, Beat, CH-4127 Birsfelden (CH); KIHLEN, Joanna, CH-4058 Basel (CH); ANDERMATT, Daniel, CH-4313 Möhlin (CH)
(74) Vertreter: Höhfeld, Jochen
(86) Internationale Anmeldenummer: PCT/IB2003/005243
(87) Internationale Veröffentlichungsnummer: WO 2005/048857

(56) Entgegenhaltungen:
- EP-A- 1 250 892
- FR-A- 2 472 373
- FR-A- 2 827 500
- GB-A- 2 245 498
- US-A1- 2003 040 749

## Beschreibung

Die Erfindung betrifft ein Osteosynthese-Set mit mindestens einer ersten und mindestens einer zweiten Osteosyntheseplatte mit insgesamt mindestens vier Bohrlöchern für Knochenschrauben.

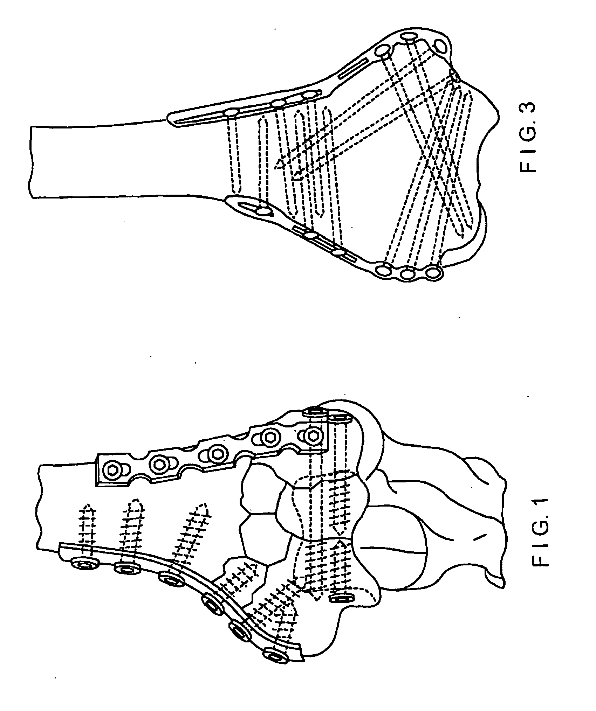

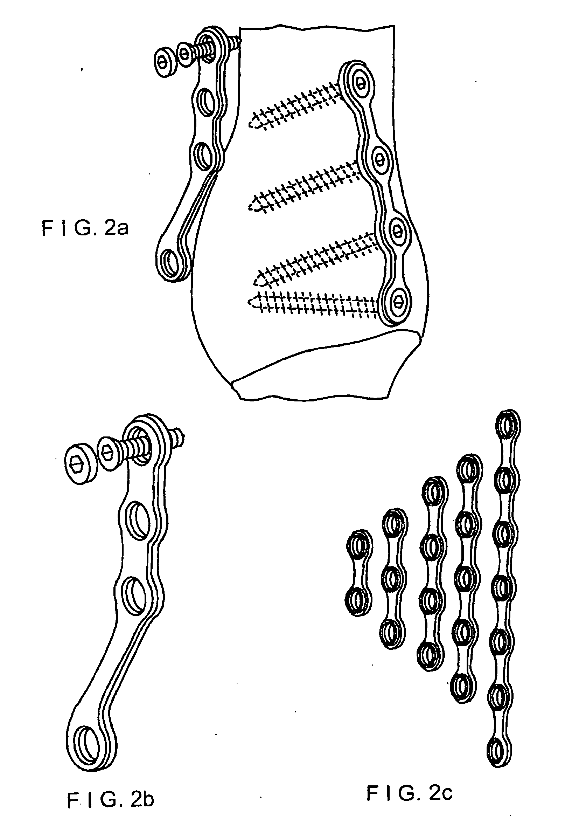

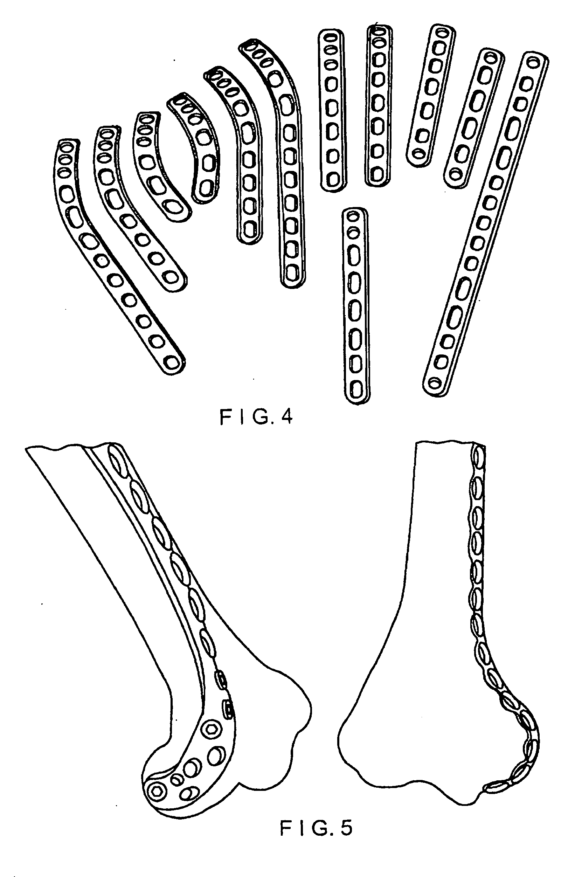

Osteosyntheseplatten zur Implantation, anatomischer Repositionen und innerer Schienung von Knochenfragmenten nach Frakturen sind weithin bekannt. Eine Knochenplatte zur Osteosynthese könnte von allgemeiner Art sein, was bedeutet, dass die Knochenplatte nicht einer bestimmten anatomischen Position angepasst wird, oder sie könnte bestimmter Art sein, was bedeutet, dass die Eigenschaften der Knochenplatte angepasst worden sind, um einer bestimmten Art einer anatomischen Position zu entsprechen. Für den Ellbogen, bzw. distalen Humerus wurden bis anhin verschiedene Lösungen angeboten (vgl. Fig. 1 bis 5);
1) Systeme mit verschiedenen Platten für eine Osteosynthese einer anatomisch nicht speziellen Art. Diese Platten wurden für beliebige anatomische Gegebenheiten erzeugt und passen so unter Umständen auch auf den distalen Humerus. Diese Knochenplatten müssen während der Operation geformt werden, um an die Anatomie ihrer entgültigen anatomischen Bestimmung angepasst zu werden, in diesem Fall an die Form des distalen Humerus.
2) Systeme von zwei an einem Knochen zusammen wirkenden Platten zur Osteosynthese, wobei eine der Platten, die mediale oder die laterale, anatomisch vorgeformt wurde, um die mediale oder die laterale Säule des distalen Humerus anzupassen. Die andere Platte ist von anatomisch nicht spezifischer Art und muss während der Operation geformt werden, um sich an ihre Lage beim distalen Humerus anzupassen.
3) Zwei-Platten-Systeme, wobei eine der Platten vorgeformt ist, um sich der Anatomie des lateralen Bogens des distalen Humerus anzupassen, und die andere Platte vorgeformt ist, um sich an den medialen Kamm des distalen Humerus anzupassen, in einer nahezu parallelen Position zueinander. Diese Platten erfordern keine oder nur wenige Umformungen während der Operation. Knochenschrauben, welche durch eine der Platten eingeführt werden, treffen in einem stumpfen Winkel auf die Knochenschrauben, welche durch die andere Platte eingeführt wurden, was als sogenannte distale Verriegelung der Schrauben bezeichnet wird.
4) Zwei-Knochenplatten-Systeme, wobei eine der Knochenplatten vorgeformt ist, um sich der Anatomie des dorsalen Aspekts des lateralen Teils anzupassen, und wobei die andere Knochenplatte vorgeformt ist, um sich an den medialen Kamm des distalen Humerus anzupassen und wobei beide Platten in einer beinahe senkrecht aufeinander stehenden Position am Humerus festgelegt werden. Diese Platten erfordern keine oder beschränkte Umformungen während der Operation. Knochenschrauben, welche durch eine der Platten eingeführt werden, treffen in einem spitzen Winkel auf die Knochenschrauben, welche durch die andere Platte eingeführt wurden.
5) Zwei-Knochenplatten-Systeme wie unter 4); ausserdem ist im entferntesten distalen Bereich der lateralen Osteosyntheseplatte die Platte nicht nur distal, sondern auch lateral erstreckt, wobei sie dort eine Bohrung aufweist. Durch diese Bohrung wird eine Knochenschraube die Knochenschrauben, welche von der medialen Platte kommen, in einem stumpfen Winkel treffen.

Im Gegensatz zu anderen Verfahren, wie blosses Eingipsen, Verschrauben mit Knochenschrauben oder Bandagieren mit Drähten haben Osteosyntheseplatten den Vorteil, mehrere Knochenfragmente zu fixieren und stabil am gesunden Gewebe festzulegen. Allen Überlegungen bei der Herstellung von Osteosyntheseplatten lag grundsätzlich die Aufgabe zugrunde, die Form dieser Osteosyntheseplatten für die Implantation am Knochen so auszubilden, dass ein anatomiegerechtes Anlegen und Fixieren der Osteosyntheseplatte ohne die Notwendigkeit von Veränderungen am Knochen und unter weitgehender Weichteilschonung erreicht wird. Die Osteosyntheseplatten sollen daher nicht voluminös sein und verschiedene Befestigungsmöglichkeiten (mehrere Bohrlöcher) aufweisen.

Aus der EP-1250892 ist ein Reparatur-System für Knochen, insbesondere für Knie bekannt, welches zwei Platten, d.h. eine Femur-Platte und eine Tibia-Platte mit Löchern aufweist. In einem der Löcher ist eine zusätzliche Buchse bewegbar angeordnet ist. Die äußere Oberfläche der Buchse und die Innenwand des Loches so ausgebildet sind, dass sie für die Buchse im Plattenloch eine polyaxiale Rotation bzw. Bewegung ermöglichen. Hier ist also die eine Platte zum Femur, die andere Platte aber zum Tibia fixiert; die Platten und die zugehörigen Schrauben sind also voneinander vollständig getrennt angeordnet, wobei die Achsen der Schrauben zufälligerweise in mehrere Raumrichtungen zeigen können.

Die FR-2827500 offenbart eine einzige T-förmige Osteosynthese-Platte zu langen Knochen, welche aus einem Längsstück und einem Querstück jeweils mit Löchern für Schrauben besteht. Diese Platte ggf. mit zusätzlichen Zungen kann zum obigen (proximal) Ende von Humerus verwendet werden.

Die GB-2245498 betrifft eine einzige Y-förmige Knochenplatte für den distalen Humerus, welche aus einem am Knochenschaft anzulegenden Hauptteil und zwei daran anschließenden Gabelteilen besteht. Die Gabelteilen und auch der Hauptteil sind mit Löchern für Schrauben versehen und gebogen.

Aus der FR-2472373 ist eine L-förmige Knochenplatte für Epiphysenfuge bekannt. Sowohl der vertikale wie auch der horizontale Arme der Platte sind mit Löchern für Schrauben versehen. In Figur ist eine solche Anordnung sichtbar, wobei zwei etwa gleiche L-förmige Knochenplatten nebeneinander am oberen Ende der Tibia fixiert sind, wobei die Achsen der Schrauben zufälligerweise in mehrere Raumrichtungen zeigen können.

James Self et al. in J. Shoulder Elbow Surg. 1995, Seiten 10-16 ("A comparison of double-plate fixation methods for complex distal humerus fractures") beschreiben verschiedene Verfahren, komplexe Frakturen des distalen Humerus zu behandeln. Beispielsweise wird erwähnt, zwei Osteosynthese-Platten zu verwenden, von denen eine auf der medialen Oberfläche, die andere auf der posterioren Oberfläche des distalen Humerus angeordnet ist. Es werden die Ergebnisse verschiedener biomechanischer Tests verglichen.

Mit den auf dem Markt erhältlichen Systemen gibt es folgende Nachteile:
1) Nicht-spezielle Verformung der Platten erfordert aufwändiges Plattenbiegen, was zu einem Verlust der mechanischen Stabilität der Platten Osteosynthese führen kann. Aufwändige Anpassung der Platten ist außerdem auch zeitraubend. Da die Platten nicht für den distalen Humerus optimiert sind, können fehlende Knochenunterstützung und Befestigungspunkte für die Knochenschrauben auftreten.
2) Aus Stabilitätsgründen wurde und wird ein Zwei-Platten-System für den distalen Humerus vorgezogen. Mit einer Platte, die geformt ist, um an einer speziellen anatomischen Stelle zu passen, und einer Platte, welche eine Anpassung an ihre Position benötigt, ist der Komfort von einem Zwei-Plattierung speziell vorgeformten Systems jedoch nicht erreicht.
3) Ein Plattensystem mit einer parallelen Plattenkonfiguration setzt nachteiligerweise voraus, dass alle Knochenfragmente etwa in einem ebenen, relativ flachen Raum zwischen den beiden Osteosyntheseplatten liegen, bzw. dort durch eingesetzte Knochenschrauben erfasst werden können. Liegen jedoch Knochenfragmente etwas außerhalb dieses relativ schmalen, ebenen Raums, so können diese durch die bekannten Osteosyntheseplatten, bzw. durch die Knochenschrauben in den bekannten Osteosyntheseplatten nicht erfasst und repositioniert gehalten werden. Außerdem beobachteten die Erfinder, dass die einreihigen Osteosyntheseplatten nur eine äußerst geringe Kippstabilität aus der verbindenden Axialebene der Bohrungen heraus aufweisen kann. Dementsprechend ist die Belastungsmöglichkeit des mit der Platte versehenen Knochens daher u. U. stark begrenzt.
4) Eine rechtwinkelige Plattenanordnung sorgt für Kippstabilität, jedoch fehlt die Möglichkeit eines stumpfwinkligen Schraubenineinandergreifens zwischen der Knochenschraube, welche durch die laterale Platte eingeführt wurde und zwischen den Knochenschrauben, welche durch die mediale Platte eingeführt Wurden. Für sehr distal gelegene Brüche und für Brüche im osteoponischen Knochen wird das Fehlen des stumpfen Zusammentreffens der Schraubenrichtungen, das sogenannte Fehlen des Zusammenschlusses über den distalen Block, den Halt der Knochenplatten im Knochen und dadurch die Stabilität der Osteosynthese herabsetzen.
5) Eine Kombination der rechtwinkeligen Plattenkonfiguration und einer lateralen Plattenform, die ein spitzwinkliges Schraubenineinandergreifen zwischen der lateralen Knochenschraube, welche durch die Patte eingeführt wurde, und der medialen Knochenschrauben, welche durch die Platte eingeführt wurden, erlaubt, ist ideal für die Stabilität und den Halt, jedoch muss die Anatomie des distalen Humerus befolgt werden. Der distale Teil der Platte, welcher lateral gestreckt wird, darf das weiche Gewebe und die Funktion des Ellbogens nicht stören. Systeme, welche auf dem Markt sind und dieser Beschreibung entsprechen, können nachteiligerweise im Bereich der Angriffsstellen der Sehnen zu liegen kommen und dadurch möglicherweise die Funktion des Ellbogens stören. Ausserdem ermöglichen auf der medialen Seite die bekannten Platten für Osteosynthese keine ausreichende Befestigung durch den distalen Block.

Der Erfindung liegt somit als Aufgabe zugrunde, die bekannten Sets zu verbessern, sodass die Osteosyntheseplatten einerseits einen besseren Halt am Knochen haben und andererseits aber mehr verschiedene Knochenfragmente sicher zueinander repositioniert werden können. Dies soll in einer weichteilschonenden Art geschehen.

Gelöst wird diese Aufgabe durch die Anwendung der Merkmale des Anspruchs 1. Durch die neuartige Ausbildung der beiden Osteosyntheseplatten kommt es zu einer vollständigeren Durchdringung des Knochengewebes mit Knochenschrauben, sodass schon mit weniger Knochenschrauben eine bessere Fixierung der Knochenfragmente erreicht werden kann. Ausserdem werden auch seitlich liegende Knochenfragmente erfasst und insgesamt eine deutlich stabilere und drehfestere Schienung möglich

Die Idee hinter der erfindungsgemässen Lösung steckt dabei in einer besonderen Aufteilung der in den Osteosyntheseplatten festlegbaren Knochenschrauben im Hinblick auf unterschiedliche Raumlagen derselben im montierten Zustand. Ein erfindungsgemässes Osteosyntheseplatten-Set ermöglicht somit auch kompliziertere Brüche zu repositionieren und darüber hinaus von Anfang an grössere Kräfte und Momente zu übertragen. Einem vorzeitigen Lockern der erfindungsgemässen Osteosyntheseplatten wird dadurch vorgebeugt. Die bevorzugte Ausbildung, bei der die Schrauben winkelstabil vorgesehen sind, unterstützt diesen erfindungsgemässen Effekt.

Eine der beiden Osteosyntheseplatten weist in ihrem Endbereich in medialer Ansicht einen dreieckförmigen Endbereich auf. Durch diese Formgebung kann diese Osteosyntheseplatte bis in den äusserst distalen Bereich des distalen Humerus verlegt werden, ohne dort Nerven, Sehnen oder dergleichen zu behindern.

Die andere der beiden Osteosyntheseplatten ist in medialer Ansicht stielförmig und mit einem davon abragenden, laschenartigen Teil ausgebildet, sodass diese Osteosyntheseplatte in medialer Ansicht etwa wie ein "P" aussieht. Da erfindungsgemäss sowohl der stielförmige als auch der laschenartige Teil Bohrlöcher trägt, ist durch diesen Aufbau eine besonders gute Winkelstabilität, aber auch ein besonders gutes Erfassen unterschiedlichster Knochenfragmente möglich. Zudem erlaubt dieser Aufbau eine sogenannte 90°-Montage der beiden Osteosyntheseplatten, bei der die Ebenen, in denen die jeweiligen Knochenschrauben liegen, etwa senkrecht zueinander stehen, wie dies durch AO vorgeschlagen wird.

Die Kreuzung der Knochenschraubenachsen der beiden Osteosyntheseplatten liegt bei dieser Ausführungsform etwa bei 90° - verglichen mit den etwa 180° bei bekannten, weichteilschonenden Systemen.

Bei einer Krümmung des laschenartigen Teiles ist die Anatomie des distalen Humerus besser berücksichtigt und zudem die Möglichkeit der Verwendung verschiedenster Raumrichtungen für die Knochenschrauben erleichtert möglich.

Als optimal hat sich eine Kombination der Osteosyntheseplatten mit dem p-förmigen, laschenartigen Teil und der Osteosyntheseplatten mit dem dreieckförmigen, distalen Bereich der Osteosyntheseplatte erwiesen. Einerseits ist der Materialbedarf gering und andererseits die Winkelstabilität verbessert, bzw. eine verbesserte Durchdringung der Knochenfragmente möglich. Erfindungsgemäß werden die beiden Osteosyntheseplatten dabei so montiert, dass die längere Seitenkante der einen Osteosyntheseplatte der laschenfreien Seitenkante der anderen Osteosyntheseplatte zugewandt ist, sodass die Lasche auf der radialen, bzw. lateralen Seite zu liegen kommt und die längere Seite der Osteosyntheseplatte mit dem dreieckförmigen Abschnitt eine längere Posterior-Kante aufweist als die Länge der Anterior-Kante.

Weiterentwicklungen der Erfindung sind in den abhängigen Ansprüchen angegeben. Dabei erzeugen die folgenden Merkmale folgende zusätzlichen Effekte: Durch eine bevorzugte Ausführung mit insgesamt mehr als vier Bohrlöchern, z.B. insgesamt acht oder zehn Bohrlöchern - wie an sich bekannt - können weitere Verbesserungen erzielt werden, wenn diese Bohrlöcher in zwei aufeinander stehenden Normalebenen betrachtet - mit ihren Bohrachsen dem Knochen zugewandt - je miteinander einen spitzen Winkel einschliessen, und es innerhalb des Systems einen intraoperativen Halt gibt, während diese oder andere mit anderen oder diesen Bohrachsen stumpfe Winkel einschliessen.

Unter "Winkel einschliessen" im Sinne der Erfindung sind nicht nur sich berührende bzw. schneidende Bohrachsen gemeint, sondern insbesondere auch Bohrachsen, die sich lediglich kreuzen, in der Ansicht von Schnittebenen jedoch scheinbar schneiden. Eine weitere Verbesserung der Winkel- und Kippstabilität von erfindungsgemässen Osteosyntheseplatten ergibt sich durch das Vorsehen von wenigstens zwei oder wenigstens drei parallelen Reihen von Bohrlöchern oder zur Wahl wenigstens einer Reihe von Bohrlöchern und dazu seitlich versetzt wenigstens ein weiteres Bohrloch.

Bevorzugt liegen die Bohrlöcher entlang zweier paralleler und dazu wenigstens zweier weiterer paralleler auf die ersten parallelen normalen Schnittebenen. Somit ist die Dreh- und Kippstabilität verbessert und darüber hinaus ist eine bessere Ausnutzung des Knochengewebes möglich, das im Randbereich eher stärker ist als in der Mitte des Humerus.

Bevorzugt werden die erfindungsgemäss verwendeten Knochenschrauben nicht nur im Knochen festgelegt, sondern auch über ein Gewinde oder Teilgewinde in den Bohrungen der Osteosyntheseplatte. Dies bewirkt vorteilhafterweise eine verbesserte Winkelstabilität der montierten Osteosyntheseplatten und reduziert die dynamische Belastung des Knochengewebes, in dem die Knochenschraube verankert ist.

Zudem bringt diese winkelstabile Ausrüstung der erfindungsgemässen Osteosyntheseplatten den Vorteil, dass selbst bei Lockerung des einen oder anderen Knochengewebes im Bereich der einen oder anderen Knochenschraube die anderen Knochenschrauben die Winkelstabilität der Osteosyntheseplatte garantieren können. Durch die Festlegung der Knochenschrauben in der Osteosyntheseplatte wird ausserdem der Anpressdruck der Osteosyntheseplatte auf den Knochen reduziert, was mithilft, druckbedingten Knochenabbau im Gewindebereich zu vermeiden. Dabei können die an sich bekannten Ausführungsformen von Knochenschrauben und Bohrlöchern vorgesehen sein.

Verfahren, bei denen die Knochenschrauben durch eine zusätzliche, lediglich in die Osteosyntheseplatte eingesetzte Druckschraube winkelstabilisiert werden, sind nach Ansicht der Erfinder insofern nicht bevorzugt, als dazu die Osteosyntheseplatte eine relativ starke Dicke aufweisen muss und die Manipulation mit den zusätzlichen, meist sehr kleinen, flachen Schrauben unter Operationsbedingungen schwierig ist.

Die Bohrungen können bei einer weiterentwickelteren Ausbildungs-Form auch so ausgelegt sein, dass Knochenschrauben in beliebigen Winkelpositionen eingedreht und winkelstabil fixiert werden können.

Ebenso für die Verbesserung der Winkelstabilität, aber auch für die Verbesserung der Kippstabilität dienen bevorzugt drei Bohrlöcher mindestens einer Osteosyntheseplatte, wobei die drei Bohrlöcher untereinander die Eckpunkte eines vorzugsweise gleichseitigen Dreieckes bilden.

Sind die Bohrungen schlüssellochförmig ausgebildet, so ergeben sich daraus die Vorteile, die bei der LCP-Platte der Anmelderin oder beispielsweise aus der WO-A-02096309 der Anmelderin bekannt geworden ist.

Weitere Entwicklungen und Details der Erfindung sind in der Figurenbeschreibung offenbart.

### Figurenbeschreibung

Die Figuren werden zusammenhängend und übergreifend beschrieben. Gleiche Bezugszeichen bedeuten gleiche Bauteile, Bezugszeichen mit unterschiedlichen Indices geben funktionsgleiche Bauteile an.

Es zeigen dabei:
- Fig.1: einen Stand der Technik mit unspezifischen Implantaten;
- Fig.2: einen anderen Stand der Technik, mit einer spezifischen und einer unspezifischen Platte;
- Fig.3: einen anderen Stand der Technik, mit zwei spezifischen Platten 180° zu einander montiert;
- Fig. 4: ein Plattensortiment aus dem Stand der Technik für Osteosynthese des distalen Humerus;
- Fig.5: einen weiteren Stand der Technik, mit zwei spezifischen Platten 90° zu einander und einem distalen, lateralgehenden Teil, zeigen einen stumpfen Winkel der von der 90° Platte abragenden Schrauben relativ zu den Schrauben der anderen Platte. Eine Fixierung der Winkellage der Schrauben ist nicht vorgesehen;
- Fig.6: eine schematische Darstellung des erfindungsgemässen Sets von Osteosyntheseplatten 2g,h in angelegtem Zustand;
- Fig.7: eine schematische Darstellung eines anderen Sets nicht erfindungsgemässer Osteosyntheseplatten 2g,i in angelegtem Zustand;
- Fig.8: eine schematische Darstellung der Osteosyntheseplatte 2g gemäss den Fig. 6 und 7 in Draufsicht;
- Fig.9: eine schematische Darstellung der Osteosyntheseplatte 2h gemäss der Fig. 6 in Draufsicht;
- Fig.10: eine schematische Darstellung der nicht erfindungsgemäßen Osteosyntheseplatten 2i gemäss der Fig. 7 in Draufsicht;
- Fig. 11: eine schematische Darstellung der Osteosyntheseplatten 2g,h in angelegtem Zustand, etwas im Vergleich zu Fig. 6 gedreht;
- Fig. 12: eine schematische Darstellung der Osteosyntheseplatten 2g,i in angelegtem Zustand, etwas im Vergleich zu Fig. 7 gedreht;
- Fig.13: eine schematische Darstellung einer einzigen Osteosyntheseplatte 2g aus dem Set in angelegtem Zustand, wobei die Osteosyntheseplatte 2g einen dreieckförmigen Endbereich 7 aufweist;
- Fig.14: eine schematische Darstellung einer einzigen Osteosyntheseplatte 2h aus dem Set in angelegtem Zustand, wobei die Osteosyntheseplatte 2h einen laschenartigen Teil 4 aufweist, und am Endbereich löffelförmig gekrümmt ist;
- Fig.15: eine schematische Darstellung einer einzigen nicht erfindungsgemäßen Osteosyntheseplatte 2i aus dem Set in angelegtem Zustand, wobei die Platte löffelartige Endbereich aufweist;
- Fig.16: eine mediale Platte mit Trochlea-Abstützung und biegbarer Verlängerung, zur Anpassung an Knochen, intraoperativ;
- Fig.17: eine Sicht dorsal auf ein Plattensystem aus medialer (ohne Trochlea-Abstützung) und lateraler Platte (mit Flansch), rechter Arm;
- Fig.18: eine Sicht frontal auf ein System gemäss Fig.17 und
- Fig.19: eine Sicht caudal auf ein System gemäss Fig.17.

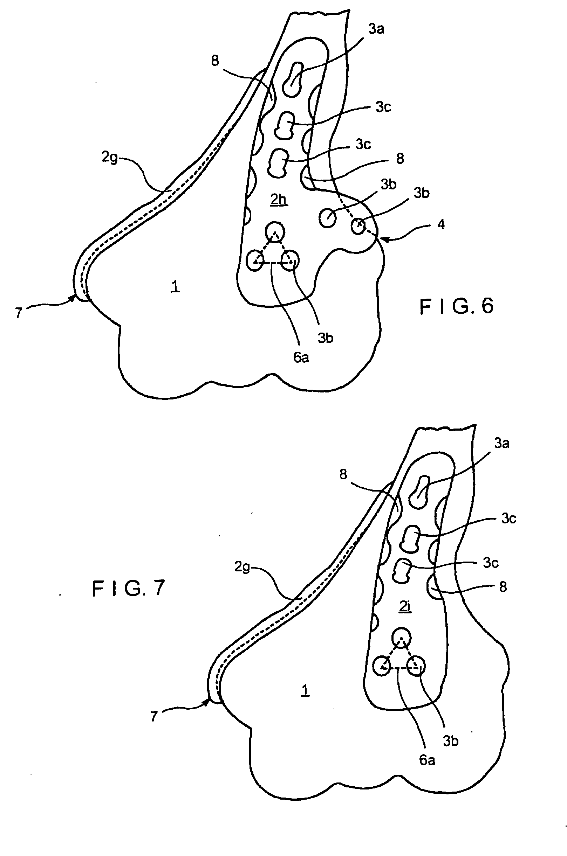

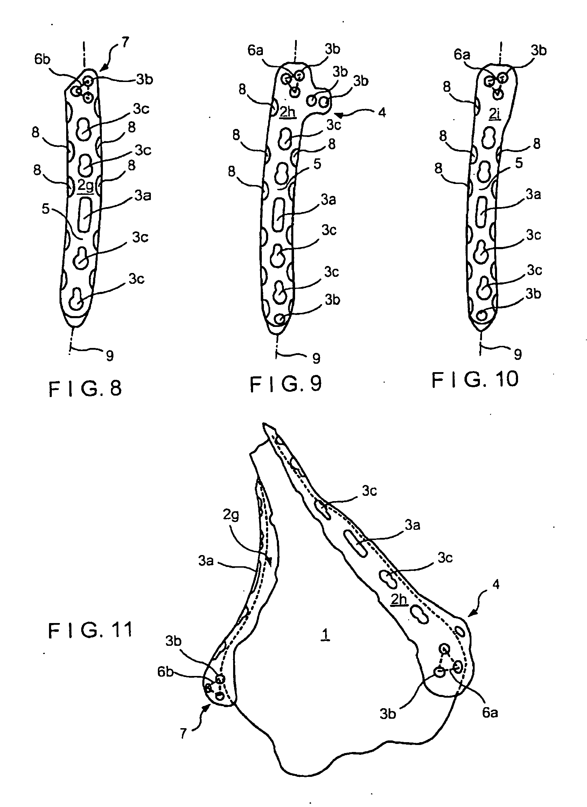

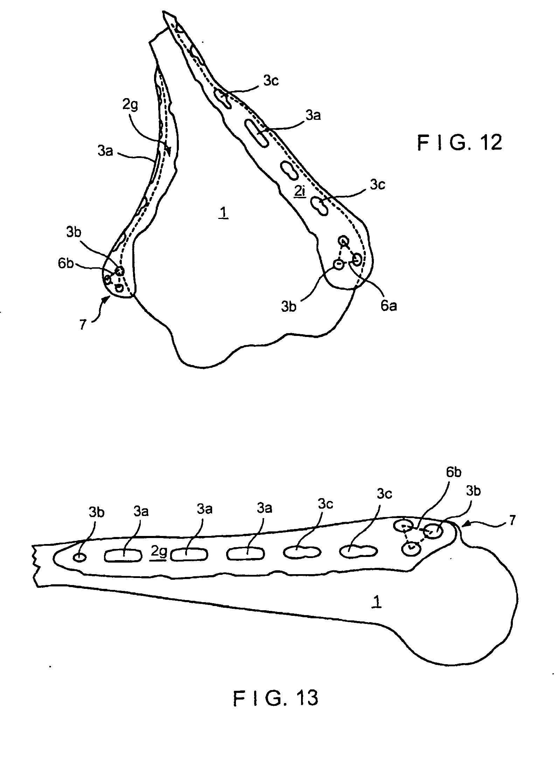

Die Fig.1-5 zeigen die heutigen auf dem Markt verfügbaren Lösungen. Die Fig. 3-5 zeigen die neuesten Lösungen auf dem Markt: den Stand der Technik gemäss Acumed und Zimmer, während die Fig. 6, und 11 erfindungsgemässe Osteosyntheseplatten - Sets 2g,h im angelegten Zustand darstellen. Fig. 7 und 12 zeigen nicht erfindungsgemäße Sets.

Die Fig.8-9 zeigen zwei verschiedene Osteosyntheseplatten 2g,h eines erfindungsgemässen Sets und Fig.10 eine nicht erfindungsgemäße Variante 2i zum Aufbau nach Fig.9 ohne Lasche.

In den Fig.6 und 7 sind verschiedene Lochformen 3a,b,c in den Osteosyntheseplatten 2h und 2i zu sehen, wie ebenso aus den Fig.6,7 und 11,12 die bevorzugte Position der Osteosyntheseplatten 2g,h,i am Knochen 1 dargestellt ist.

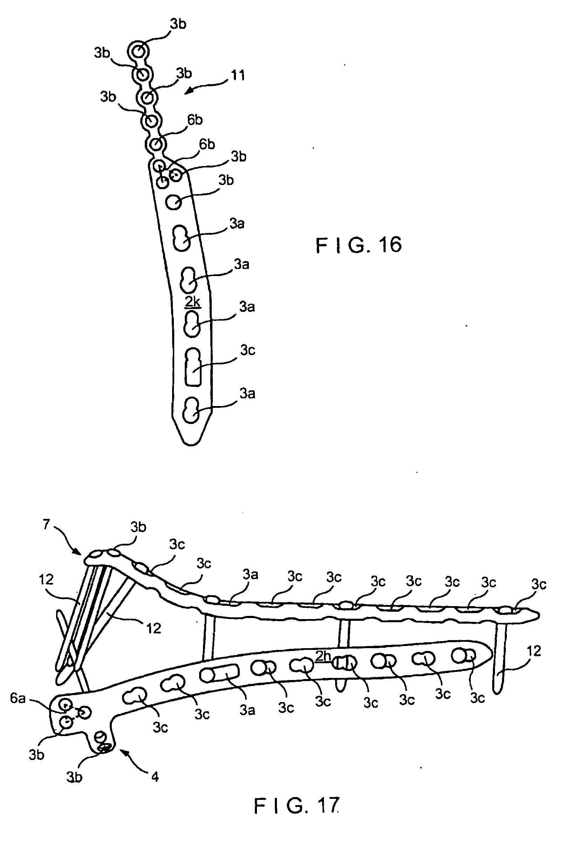

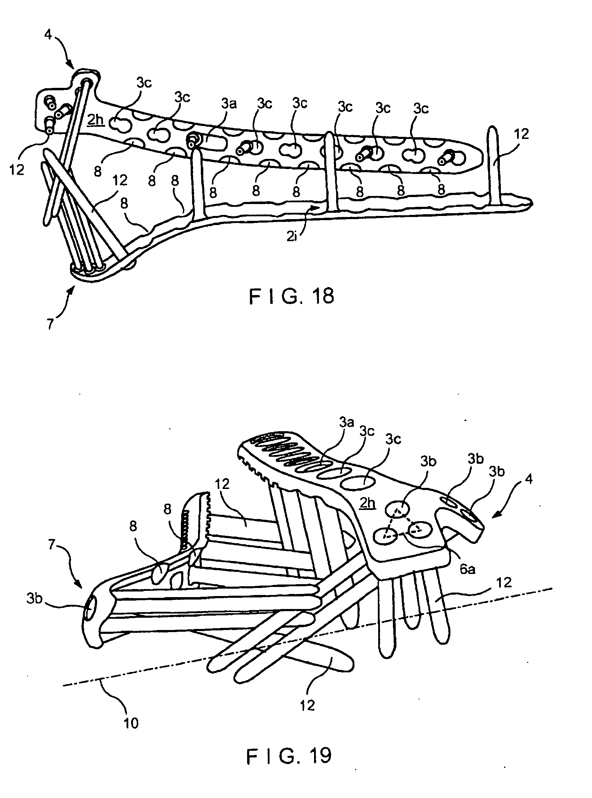

Wie aus den Darstellungen in den Fig.6 und 11 ersichtlich, liegen Bohrlöcher 3a,b,c entlang von Achsen, die sich mit vergleichbaren Achsen der Bohrlöcher 3a,b,c der jeweils anderen Osteosyntheseplatte 2h,g etwa rechtwinkelig kreuzen. Darüber hinaus befinden sich im Bereich des laschenartigen Teils 4 weitere Bohrungen 3b, die in Folge der laschenartigen Krümmung der Flansche 4 nicht parallel zu den Achsen der übrigen Bohrungen 3a,b,c derselben Osteosyntheseplatte 2h liegen. Bei der gedanklichen Ansicht eines Sets mit Knochenschrauben 12 gemäss Fig.17-19 erkennt man, dass diese nun nicht mehr nur vor einem schmalen, ebenen Bereich zu liegen kommen, sondern den Knochenraum in verschiedensten Raumrichtungen in spitzen und stumpfen Winkeln zueinander durchdringen und derart besser geeignet sind, verschieden positionierte Knochenfragmente zu repositionieren und zu schienen.

In Fig.8-10 sowie ansatzweise in Fig.6-7 und 11-12 sind im bohrlochfreien Bereich der Osteosyntheseplatten 2g,h fingerkuppenartige Unterschnitte 8 dargestellt, die entweder wie dargestellt nur Materialschwächungen sind oder auch vollständig freigestellte (ausgefräste) Bereiche symbolisch darstellen. Diese Unterschnitte 8 dienen dazu, Störungen der Platte am Knochen zu verringern, indem da der Kontakt zwischen Knochen und Platte minimiert ist. Zudem wird das Gewicht der Platten dadurch reduziert.

Ebenso sind die an sich bekannten unterschiedlichen Formen der Bohrlöcher 3a,b,c wie langlochförmige Bohrungen 3a, schlüssellochförmige Kombinationsbohrungen 3c und runde Bohrungen 3b zu sehen, wobei wenigstens ein Teil der erfindungsgemässen Bohrungen 3a,b,c über ein Innengewinde verfügt, das mit den Knochenschrauben 12 winkelstabilisierend zusammenwirkt.

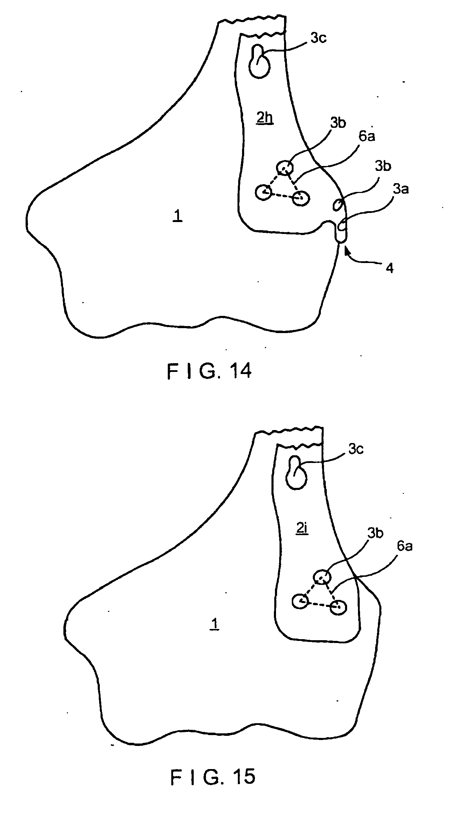

Die Fig.13-15 zeigen je eine Osteosyntheseplatte 2g,h,i in angelegtem Zustand, wobei Fig.13 eine Osteosyntheseplatte 2g mit einem dreieckförmigen Endbereich 7, Fig.14 eine Osteosyntheseplatte 2h mit einem laschenartigen Teil 4 zeigt, der gekrümmt ist und einen löffelartigen Endbereich aufweist und Fig.15 eine Osteosyntheseplatte 2i, die einen löffelartigen Endbereich aufweist.

### Bezugszeichenliste

- 1: Knochen
- 2g: Erste Osteosyntheseplatte
- 2h: Zweite Osteosyntheseplatte
- 2i: Dritte Osteosyntheseplatte
- 2k: Vierte Osteosyntheseplatte
- 3a: Langlochförmige Bohrung
- 3b: Runde Bohrung
- 3c: Locking-Compression Kombinationsloch
- 3d: Runde Bohrung
- 4: Laschenartiger Teil, Flansch
- 5: Stielförmiger Teil
- 6a: Dreieck
- 6b: Dreieck
- 7: Dreieckförmiger Endbereich
- 8: Unterschnitte
- 9: Zentralachse
- 10: Gelenkachse
- 11: Verschmälerte Verlängerung
- 12: Knochenschraube

## Patentansprüche

1. Implantierbares Osteosynthese-Set zum Schienen und Verschrauben eines Knochens (1) mit mindestens einem Gelenk und einer Gelenkachse (10), insbesondere für einen distalen Humerus,
wobei das Set enthält:
- mindestens eine erste Osteosyntheseplatte (2g), die angepasst ist, an einem Knochen (1) im Bereich eines Gelenks, insbesondere an einem distalen Humerus, an einer ersten Stelle implantiert zu werden,
- mindestens eine zweite Osteosyntheseplatte (2h, 2i,...), die angepasst ist, an demselben Knochen (1) im Bereich des Gelenks an einer zweiten Stelle implantiert zu werden, und
- insgesamt mindestens vier, die Fläche der Osteosyntheseplatten (2g, 2h, 2i,...) durchsetzende Löcher (3a, 3b, 3c) für Knochenschrauben (12), deren Achsen im implantierten Zustand in mehrere Raumrichtungen zeigen, wobei die mindestens zwei Osteosyntheseplatten (2g, 2h, 2i) und/oder deren insgesamt mindestens vier Löcher (3a, 3b, 3c) so ausgebildet und eingerichtet sind, dass im implantierten Zustand die Achsen der Löcher (3a, 3b, 3c) in mindestens vier verschiedene Raumrichtungen zeigen, wobei mindestens eine der mindestens zwei Osteosyntheseplatten (2g, 2h, 2i) mindestens zwei der Löcher (3a, 3b, 3c) für Knochenschrauben (12) aufweist, die derart eingerichtet sind, dass deren Achsen im implantierten Zustand mit der Gelenkachse (10) einen spitzen Winkel einschließen, wobei mindestens eines der Löcher (3a, 3b, 3c) derart eingerichtet ist, dass dessen Achse im implantierten Zustand zu der Gelenkachse (10) wenigstens nahezu rechtwinkelig angeordnet ist, **dadurch gekennzeichnet,**
**dass** eine der Osteosyntheseplatten (2g, 2h) in Draufsicht einen stielförmigen Teil (5) und einen davon abragenden laschenartigen Teil (4) aufweist, wobei sowohl der stielförmige Teil (5) als auch der laschenartige Teil (4) mit Löchern (3a, 3b, 3c) versehen ist, und dass die andere der
Osteosyntheseplatten (2g, 2h) einen, in Draufsicht dreieckförmigen, im implantierten Zustand dem Gelenk zugewandten Endbereich (7) aufweist, derart, dass eine kurzere und eine längere Seitenkante die anderen Osteosyntheseplatte (29, 24) ausgebildet ist,
wobei die beiden Osteosyntheseplatten (2g, 2h) derart eingerichtet sind, dass sie mit dem dreieckförmigen Endbereich (7) bzw. mit dem laschenartigen Teil (4) zueinander so anordenbar sind, dass die längere
Seitenkante der einen Osteosyntheseplatte (2g; 2h) einer laschenfreien Seitenkante der anderen Osteosyntheseplatte (2h; 2g) zugewandt ist.

2. Das Osteosynthese-Set nach Anspruch 1, **dadurch gekennzeichnet, dass** zur winkelstabilen Verankerung der Knochenschraube (12) im Knochen (1) wenigstens einem der Löcher (3a, 3b, 3c) der Osteosyntheseplatten (2g, 2h, 2i) wenigstens eine Gewindebohrung zugeordnet ist, welche mit einem Kopfgewinde der Knochenschraube (12) verriegelnd verbindbar ist.

3. Das Osteosynthese-Set nach Anspruch 2, **dadurch gekennzeichnet, dass** der Winkel der winkelstabilen Verankerung im Loch (3a, 3b, 3c) wählbar ist, wozu die mindestens eine Gewindebohrung im Loch (3a, 3b, 3c) als nicht-starres Gewinde oder aus wenigstens zwei, voneinander unterschiedlichen, starren Gewinden ausgebildet ist.

4. Das Osteosynthese-Set nach Anspruch 2 oder 3, **dadurch gekennzeichnet, dass** zur winkelstabilen Verankerung im Knochen (1) jede der Osteosyntheseplatten (2g, 2h, 2i) wenigstens ein Loch, vorzugsweise wenigstens zwei Löcher (3a, 3b, 3c) mit Gewindebohrung aufweist.

5. Das Osteosynthese-Set nach einem der Ansprüche 2-4, **dadurch gekennzeichnet, dass** wenigstens vier Gewindebohrungen vorgesehen sind, wobei deren Gewindeachsen so ausgerichtet sind, dass der Winkel zwischen jeder Gewindeachse und der Plattenoberfläche bzw. zwischen jeder Gewindeachse und allen Tangenten auf die Plattenoberfläche mit einem gemeinsamen Schnittpunkt an der Gewindeachse ungleich 90° ist.

6. Das Osteosynthese-Set nach einem der Ansprüche 2-5, **dadurch gekennzeichnet, dass** wenigstens zwei von den Löchern (3a, 3b, 3c) oder wenigstens zwei von den Gewindebohrungen voneinander verschiedene Durchmesser aufweisen.

7. Das Osteosynthese-Set nach einem der Ansprüche 2-6, welches mit Knochenschrauben (12) versehen ist, die ein Gewinde für den Knochen (1) und ein Kopfgewinde haben, **dadurch gekennzeichnet, dass** das Verhältnis eines Durchmessers des Gewindes zu einem Durchmesser des Kopfgewindes der Knochenschraube (12) so klein wie möglich ist, so dass ein Durchmesser der Gewindebohrung in der Osteosyntheseplatte (2g, 2h, 2i) unwesentlich größer als der Durchmesser des Gewindes der Knochenschraube (12) für den Knochen ist.

8. Das Osteosynthese-Set nach einem der vorhergehenden Ansprüche, **dadurch gekennzeichnet, dass** mehr als insgesamt vier Löcher (3a, 3b, 3c) vorgesehen sind, und dass im implantierten Zustand die mindestens vier verschiedenen Raumrichtungen der Achsen der Löcher (3a, 3b, 3c) bzw. die eingesetzten Knochenschrauben (12) mit der Gelenkachse (10) jeweils einen spitzen Winkel einschließen, wobei der Winkel jeweils in einer Betrachtungsebene parallel zur Gelenkachse (10) gemessen ist, sodass mit der Gelenkachse (10) schneidende oder kreuzende Raumrichtungen davon erfasst sind.

9. Das Osteosynthese-Set nach einem der vorhergehenden Ansprüche, **dadurch gekennzeichnet, dass** in jeder Osteosyntheseplatte (2g, 2h) die Löcher (3a, 3b, 3c) entlang zweier paralleler und dazu wenigstens zweier weiterer, auf die Parallelen normalen Schnittebenen durch jede Osteosyntheseplatte (2g, 2h) liegen.

10. Das Osteosynthese-Set nach Anspruch 3, **dadurch gekennzeichnet, dass** die Löcher (3a, 3b, 3c) in wenigstens drei parallelen und wenigstens zwei darauf normalen Schnittebenen durch jede Osteosyntheseplatte (2g, 2h) liegen.

11. Das Osteosynthese-Set nach einem der vorhergehenden Ansprüche, **dadurch gekennzeichnet, dass** wenigstens drei von den Löchern (3b) in wenigstens einer der Osteosyntheseplatten (2g, 2h) die Eckpunkte eines vorzugsweise gleichseitigen Dreiecks (6a; 6b) bilden.

12. Das Osteosynthese-Set nach einem der vorhergehenden Ansprüche, **dadurch gekennzeichnet, dass** der laschenartige Teil (4) gekrümmt ausgebildet ist.

13. Das Osteosynthese-Set nach einem der vorhergehenden Ansprüche, **dadurch gekennzeichnet, dass** der distale Bereich wenigstens einer der Osteosyntheseplatten (2h; 2i) löffelartig gekrümmt ist.

14. Das Osteosynthese-Set nach einem der vorhergehenden Ansprüche, **dadurch gekennzeichnet, dass** wenigstens eine der Osteosyntheseplatten (2g, 2h) eine Dicke im Bereich von 1,7 - 2,7 mm, vorzugsweise 1,8 - 2,5 mm aufweist.

15. Das Osteosynthese-Set nach einem der vorhergehenden Ansprüche, **dadurch gekennzeichnet, dass** der stielförmige Teil (5) wenigstens einer der Osteosyntheseplatten (2g, 2h) mindestens in einem Teilbereich in Richtung ihrer Zentralachse (9) gesehen gekrümmt ausgebildet ist.

16. Das Osteosynthese-Set nach einem der vorhergehenden Ansprüche, **dadurch gekennzeichnet, dass** wenigstens eine der Osteosyntheseplatten (2g, 2h) mindestens in einem Teilbereich eine quer zu ihrer Zentralachse (9) verlaufende Krümmung aufweist, vorzugsweise mit einem Krümmungsradius im Bereich von 18 bis 22 mm.

17. Das Osteosynthese-Set nach einem der vorhergehenden Ansprüche, **dadurch gekennzeichnet, dass** wenigstens eine der Osteosyntheseplatten (2g, 2h) über ihre gesamte Länge eine quer zur Zentralachse (9) verlaufende Krümmung - vorzugsweise mit unterschiedlichen Krümmungsradien - verfügt.

18. Das Osteosynthese-Set nach einem der vorhergehenden Ansprüche, **dadurch gekennzeichnet, dass** in wenigstens einer der Osteosyntheseplatten (2g, 2h) wenigstens zwei Löcher (3a, 3b, 3c) in zwei zueinander annähernd parallelen Ebenen liegen, die in einem von senkrecht abweichenden Winkel auf die Oberfläche oder einer Tangente auf die Oberfläche der Osteosyntheseplatten (2g, 2h) im Schnittbereich mit jeder Ebene liegen.

19. Das Osteosynthese-Set nach einem der vorhergehenden Ansprüche, **dadurch gekennzeichnet, dass** wenigstens eines der Löcher schlüssellochförmig (3c) ausgebildet ist.

20. Das Osteosynthese-Set nach einem der vorhergehenden Ansprüche, **dadurch gekennzeichnet, dass** die Dicke und/oder Breite wenigstens einer der Osteosyntheseplatten (2g, 2h) im lochfreien Bereich (8) geringer als im Bereich der Löcher (3a, 3b, 3c) ist.

21. Das Osteosynthese-Set nach einem der vorhergehenden Ansprüche, **dadurch gekennzeichnet, dass** wenigstens eine der Osteosyntheseplatten (2g, 2h) an ihrem der Gelenkachse (10) zugewandten Ende eine verschmälerte Verlängerung (11) aufweist, die wenigstens ein Loch (3b) für eine Knochenschraube (12) aufweist und um den Knochen (1) herum biegbar ausgebildet ist.

22. Das Osteosynthese-Set nach Anspruch 21, **dadurch gekennzeichnet, dass** die Verlängerung (11) mehrere Löcher (3b) umfasst, und die Verlängerung (11) zwischen den Bereichen mit den Löchern (3b) schmäler als im Bereich der Löcher (3b) ist, so dass die Verlängerung (11) zur Anpassung an den Knochen auch in der Plattenebene biegbar ausgebildet ist.

23. Das Osteosynthese-Set nach einem der vorhergehenden Ansprüche, welches mit Knochenschrauben (12) versehen ist, **dadurch gekennzeichnet, dass** die mindestens zwei Osteosyntheseplatten (2g, 2h) eine längliche Erstreckung entlang je einer Längsachse (9) haben; und dass im implantierten Zustand die mindestens zwei Osteosyntheseplatten (2g, 2h) in je einer, auf die andere wenigstens annähernd senkrecht stehenden Ebene angeordnet sind.

## Claims

1. An implantable osteosynthesis set for splinting and screwing together a bone (1) having at least one joint and one joint axis (10), in particular for a distal humerus, wherein the set contains:
- at least a first osteosynthesis plate (2g) which is adapted to be implanted on a bone (1) in the region of a joint, in particular on a distal humerus, at a first place,
- at least a second osteosynthesis plate (2h, 2i, ...) which is adapted to be implanted on the same bone (1) in the region of the joint at a second place, and
- altogether at least four holes (3a, 3b, 3c) for bone screws (12), which penetrate the surface of the osteosynthesis plates (2g, 2h, 2i, ...) and whose axes point in several spatial directions in the implanted state,
wherein the at least two osteosynthesis plates (2g, 2h, 2i) and/or their altogether at least four holes (3a, 3b, 3c) are so formed and configured that the axes of the holes (3a, 3b, 3c) point in at least four different spatial directions in the implanted state,
wherein at least one of the at least two osteosynthesis plates (2g, 2h, 2i) has at least two of the holes (3a, 3b, 3c) for bone screws (12), which are configured such that their axes enclose an acute angle with the joint axis (10) in the implanted state,
wherein at least one of the holes (3a, 3b, 3c) is configured such that its axis is arranged at least nearly at right angles to the joint axis (10) in the implanted state,
**characterized in that** one of the osteosynthesis plates (2g, 2h) has in plan view a stem-shaped portion (5) and a tab-like part (4) protruding therefrom, wherein both the stem-shaped portion (5) and the tab-like portion (4) are provided with holes (3a, 3b, 3c), and that the other of the osteosynthesis plates (2g, 2h) has an end region (7) that is triangular in plan view and faces the joint in the implanted state, such that a shorter and a longer side edge of the other osteosynthesis plate (2g, 2h) is formed,
wherein the two osteosynthesis plates (2g, 2h) are configured such that they are so arrangeable relative to each other with the triangular end region (7) and with the tab-like portion (4), respectively, that the longer side edge of the one osteosynthesis plate (2g; 2h) faces a tab-free side edge of the other osteosynthesis plate (2h; 2g).

2. The osteosynthesis set according to claim 1, **characterized in that** for stable-angle anchoring of the bone screw (12) in the bone (1), there is associated with at least one of the holes (3a, 3b, 3c) of the osteosynthesis plates (2g, 2h, 2i) at least one threaded bore which is lockingly connectable to a head thread of the bone screw (12).

3. The osteosynthesis set according to claim 2, **characterized in that** the angle of the stable-angle anchoring in the hole (3a, b, c) is selectable, for which purpose the at least one threaded bore in the hole (3a, 3b, 3c) is formed as a non-rigid thread or from at least two, mutually different, rigid threads.

4. The osteosynthesis set according to claim 2 or 3, **characterized in that** for stable-angle anchoring in the bone (1) each of the osteosynthesis plates (2g, 2h, 2i) has at least one hole, preferably at least two holes (3a, 3b, 3c) with a threaded bore.

5. The osteosynthesis set according to any of claims 2-4, **characterized in that** at least four threaded bores are provided, wherein their thread axes are so oriented that the angle between each thread axis and the plate surface or between each thread axis and all tangents to the plate surface with a common intersection point on the thread axis is non-equal to 90°.

6. The osteosynthesis set according to any of claims 2-5, **characterized in that** at least two of the holes (3a, 3b, 3c) or at least two of the threaded bores have mutually different diameters.

7. The osteosynthesis set according to any of claims 2-6 which is provided with bone screws (12) which have a thread for the bone (1) and a head thread, **characterized in that** the ratio of a diameter of the thread to a diameter of the head thread of the bone screw (12) is as small as possible, so that a diameter of the threaded bore in the osteosynthesis plate (2g, 2h, 2i) is negligibly greater than the diameter of the thread of the bone screw (12) for the bone.

8. The osteosynthesis set according to any of the preceding claims, **characterized in that** more than altogether four holes (3a, 3b, 3c) are provided, and that in the implanted state the at least four different spatial directions of the axes of the holes (3a, 3b, 3c) or the inserted bone screws (12) respectively enclose an acute angle with the joint axis (10), wherein the angle is respectively measured in a viewing plane parallel to the joint axis (10), so that spatial directions which intersect or cross the joint axis (10) are covered thereby.

9. The osteosynthesis set according to any of the preceding claims, **characterized in that** in each osteosynthesis plate (2g, 2h) the holes (3a, 3b, 3c) lie along two parallel sectional planes through each osteosynthesis plate (2g, 2h) and in addition at least two further sectional planes normal to the parallels.

10. The osteosynthesis set according to claim 3, **characterized in that** the holes (3a, 3b, 3c) lie in at least three parallel sectional planes through each osteosynthesis plate (2g, 2h) and at least two sectional planes normal thereto.

11. The osteosynthesis set according to any of the preceding claims, **characterized in that** at least three of the holes (3b) in at least one of the osteosynthesis plates (2g, 2h) form the corner points of a preferably equilateral triangle (6a; 6b).

12. The osteosynthesis set according to any of the preceding claims, **characterized in that** the tab-like portion (4) is curved.

13. The osteosynthesis set according to any of the preceding claims, **characterized in that** the distal region of at least one of the osteosynthesis plates (2h; 2i) is curved in spoon-like manner.

14. The osteosynthesis set according to any of the preceding claims, **characterized in that** at least one of the osteosynthesis plates (2g, 2h) has a thickness in the range of 1.7 - 2.7 mm, preferably 1.8 - 2.5 mm.

15. The osteosynthesis set according to any of the preceding claims, **characterized in that** the stem-shaped portion (5) of at least one of the osteosynthesis plates (2g, 2h) is curved at least in a partial region, viewed in the direction of its central axis (9).

16. The osteosynthesis set according to any of the preceding claims, **characterized in that** at least one of the osteosynthesis plates (2g, 2h) has, at least in a partial region, a curvature running transversely to its central axis (9), preferably with a radius of curvature in the range of 18 to 22 mm.

17. The osteosynthesis set according to any of the preceding claims, **characterized in that** at least one of the osteosynthesis plates (2g, 2h) has, over its total length, a curvature running transversely to the central axis (9), preferably with different radii of curvature.

18. The osteosynthesis set according to any of the preceding claims, **characterized in that** in at least one of the osteosynthesis plates (2g, 2h) at least two holes (3a, 3b, 3c) lie in two mutually approximately parallel planes which lie at an angle deviating from perpendicular to the surface or a tangent to the surface of the osteosynthesis plates (2g, 2h) in the intersecting region with each plane.

19. The osteosynthesis set according to any of the preceding claims, **characterized in that** at least one of the holes is keyhole-shaped (3c).

20. The osteosynthesis set according to any of the preceding claims, **characterized in that** the thickness and/or width of at least one of the osteosynthesis plates (2g, 2h) is smaller in the hole-free region (8) than in the region of the holes (3a, 3b, 3c).

21. The osteosynthesis set according to any of the preceding claims, **characterized in that** at least one of the osteosynthesis plates (2g, 2h) has a narrowed extension (11) at its end facing the joint axis (10), which has at least one hole (3b) for a bone screw (12) and is formed to be bendable around the bone (1).

22. The osteosynthesis set according to claim 21, **characterized in that** the extension (11) comprises several holes (3b), and the extension (11) is narrower between the regions with the holes (3b) than in the region of the holes (3b), so that the extension (11) is formed to be bendable also in the plate plane for adaptation to the bone.

23. The osteosynthesis set according to any of the preceding claims which is provided with bone screws (12), **characterized in that** the at least two osteosynthesis plates (2g, 2h) have an elongate extension along one longitudinal axis (9) respectively; and that in the implanted state the at least two osteosynthesis plates (2g, 2h) are respectively arranged in one plane that is at least approximately perpendicular to the other.

## Revendications

1. Kit d'ostéosynthèse implantable destiné à la mise sous attelle et fixation par vis d'un os (1) comprenant au moins une articulation et un axe d'articulation (10), notamment pour un humérus distal, le kit comprenant :
- au moins une première plaque d'ostéosynthèse (2g) qui est adaptée pour être implantée à un premier endroit à un os (1) dans la zone d'une articulation, notamment à un humérus distal,
- au moins une seconde plaque d'ostéosynthèse (2h, 2i, ...) qui est adaptée pour être implantée à un second endroit au même os (1) dans la zone de l'articulation, et
- en tout au moins quatre trous (3a, 3b, 3c) traversant la surface des plaques d'ostéosynthèse (2g, 2h, 2i, ...) pour vis d'ostéosynthèse (12), dont les axes sont orientés à l'état implanté dans plusieurs directions spatiales, les au moins deux plaques d'ostéosynthèse (2g, 2h, 2i) et/ou leurs au moins quatre trous (3a, 3b, 3c) en tout étant réalisés et configurés de telle manière que, à l'état implanté, les axes des trous (3a, 3b, 3c) sont orientés dans au moins quatre différentes directions spatiales, au moins une des au moins deux plaques d'ostéosynthèse (2g, 2h, 2i) présentant au moins deux des trous (3a, 3b, 3c) pour vis d'ostéosynthèse (12) qui sont configurés de telle façon que leurs axes incluent à l'état implanté un angle aigu avec l'axe d'articulation (10), au moins un des trous (3a, 3b, 3c) étant configuré de telle façon que son axe est agencé à l'état implanté au moins presque perpendiculairement à l'axe d'articulation (10), **caractérisé en ce qu'**une des plaques d'ostéosynthèse (2g, 2h) présente en vue de dessus une partie en forme de tige (5) et une partie en forme de patte (4) qui en dépasse, tant la partie en forme de tige (5) que la partie en forme de patte (4) étant pourvue de trous (3a, 3b, 3c), et **en ce que** l'autre des plaques d'ostéosynthèse (2g, 2h) présente une partie terminale (7) en forme de triangle en vue de dessus, tournée vers l'articulation à l'état implanté, de telle façon qu'une arête latérale plus courte et un plus longue de l'autre plaque d'ostéosynthèse (2g, 2h) est réalisée, les deux plaques d'ostéosynthèse (2g, 2h) étant configurées de telle manière qu'elles peuvent être agencées de telle façon l'une par rapport à l'autre avec la partie terminale (7) en forme de triangle ou avec la partie en forme de patte (4) que l'arête latérale plus longue de la une plaque d'ostéosynthèse (2g; 2h) est tournée vers une arête latérale sans patte de l'autre plaque d'ostéosynthèse (2h; 2g).

2. Le kit d'ostéosynthèse selon la revendication 1, **caractérisé en ce que**, pour l'ancrage angulairement stable de la vis d'ostéosynthèse (12) dans l'os (1), il est affecté à au moins un des trous (3a, 3b, 3c) des plaques d'ostéosynthèse (2g, 2h, 2i) au moins un alésage fileté qui peut être relié avec une tête filetée de la vis d'ostéosynthèse (12) de façon à assurer un verrouillage.

3. Le kit d'ostéosynthèse selon la revendication 2, **caractérisé en ce que** l'angle de l'ancrage angulairement stable dans le trou (3a, 3b, 3c) peut être choisi, le au moins un alésage fileté dans le trou (3a, 3b, 3c) étant réalisé à cet effet sous forme de filetage non rigide ou composé d'au moins deux filetages rigides différents l'un de l'autre.

4. Le kit d'ostéosynthèse selon la revendication 2 ou 3, **caractérisé en ce que**, pour l'ancrage angulairement stable dans l'os (1), chacune des plaques d'ostéosynthèse (2g, 2h, 2i) présente au moins un trou, de préférence au moins deux trous (3a, 3b, 3c) pourvus d'alésage fileté.

5. Le kit d'ostéosynthèse selon une des revendications 2-4, **caractérisé en ce qu'**au moins quatre alésages filetés sont prévus, dont les axes de filetage sont alignés de telle façon que l'angle entre chaque axe de filetage et la surface de la plaque ou entre chaque axe de filetage et toutes les tangentes à la surface de la plaque ayant un point d'intersection commun à l'axe de filetage est différent de 90°.

6. Le kit d'ostéosynthèse selon une des revendications 2-5, **caractérisé en ce qu'**au moins deux des trous (3a, 3b, 3c) ou au moins deux des alésages filetés présentent des diamètre différents l'un de l'autre.

7. Le kit d'ostéosynthèse selon une des revendications 2-6, qui est pourvu de vis d'ostéosynthèse (12) qui ont un filetage pour l'os (1) et une tête filetée, **caractérisé en ce que** la relation d'un diamètre du filetage par rapport à un diamètre de la tête filetée de la vis d'ostéosynthèse (12) est aussi faible que possible, de telle sorte qu'un diamètre de l'alésage fileté dans la plaque d'ostéosynthèse (2g, 2h, 2i) n'est que faiblement supérieur au diamètre du filetage de la vis d'ostéosynthèse (12) pour l'os.

8. Le kit d'ostéosynthèse selon une des revendications précédentes, **caractérisé en ce que** plus de quatre trous en tout sont prévus (3a, 3b, 3c), et **en ce qu'**à l'état implanté les au moins quatre directions spatiales différentes des axes des trous (3a, 3b, 3c) ou les vis d'ostéosynthèse (12) utilisées incluent respectivement un angle aigu par rapport à l'axe d'articulation (10), l'angle étant mesuré respectivement dans un plan d'observation parallèle à l'axe d'articulation (10), de telle sorte que des directions spatiales se coupant ou se croisant avec l'axe d'articulation (10) sont ici relevées.

9. Le kit d'ostéosynthèse selon une des revendications précédentes, **caractérisé en ce que**, dans chaque plaque d'ostéosynthèse (2g, 2h), les trous (3a, 3b, 3c) se trouvent le long de deux plans d'intersection parallèles et en plus de cela au moins le long de deux autres plans d'intersection perpendiculaires aux parallèles à travers chaque plaque d'ostéosynthèse (2g, 2h).

10. Le kit d'ostéosynthèse selon la revendication 3, **caractérisé en ce que** les trous (3a, 3b, 3c) se trouvent dans au moins trois plans d'intersection parallèles et dans au moins deux y étant perpendiculaires à travers chaque plaque d'ostéosynthèse (2g, 2h).

11. Le kit d'ostéosynthèse selon une des revendications précédentes, **caractérisé en ce qu'**au moins trois des trous (3b) dans au moins une des plaques d'ostéosynthèse (2g, 2h) constituent les points d'angle d'un triangle (6a; 6b) dont les côtés sont de préférence égaux.

12. Le kit d'ostéosynthèse selon une des revendications précédentes, **caractérisé en ce que** la partie en forme de patte (4) est réalisée sous forme courbée.

13. Le kit d'ostéosynthèse selon une des revendications précédentes, **caractérisé en ce que** la zone distale d'au moins une des plaques d'ostéosynthèse (2h; 2i) est courbée en forme de cuillère.

14. Le kit d'ostéosynthèse selon une des revendications précédentes, **caractérisé en ce qu'**au moins une des plaques d'ostéosynthèse (2g, 2h) présente une épaisseur de l'ordre de 1,7 - 2,7 mm, de préférence de 1,8 - 2,5 mm.

15. Le kit d'ostéosynthèse selon une des revendications précédentes, **caractérisé en ce que** la partie en forme de tige (5) d'au moins une des plaques d'ostéosynthèse (2g, 2h) est réalisée sous forme courbée au moins dans une zone partielle vu en direction de son axe central (9).

16. Le kit d'ostéosynthèse selon une des revendications précédentes, **caractérisé en ce qu'**au moins une des plaques d'ostéosynthèse (2g, 2h) présente au moins dans une zone partielle une courbure s'étendant transversalement à son axe central (9), de préférence avec un rayon de courbure de l'ordre de 18 à 22 mm.

17. Le kit d'ostéosynthèse selon une des revendications précédentes, **caractérisé en ce qu'**au moins une des plaques d'ostéosynthèse (2g, 2h) dispose sur toute sa longueur d'une courbure s'étendant transversalement à l'axe central (9) - de préférence avec différents rayons de courbure.

18. Le kit d'ostéosynthèse selon une des revendications précédentes, **caractérisé en ce que**, au moins dans une des plaques d'ostéosynthèse (2g, 2h), au moins deux trous (3a, 3b, 3c) se trouvent dans deux plans approximativement parallèles entre eux qui se trouvent dans un angle non perpendiculaire à la surface ou dans une tangente à la surface des plaques d'ostéosynthèse (2g, 2h) dans la zone d'intersection avec chaque plan.

19. Le kit d'ostéosynthèse selon une des revendications précédentes, **caractérisé en ce qu'**au moins un des trous est réalisé en forme de trou de serrure (3c).

20. Le kit d'ostéosynthèse selon une des revendications précédentes, **caractérisé en ce que** l'épaisseur et/ou la largeur d'au moins une des plaques d'ostéosynthèse (2g, 2h) est plus faible dans la zone sans trou (8) que dans la zone des trous (3a, 3b, 3c).

21. Le kit d'ostéosynthèse selon une des revendications précédentes, **caractérisé en ce qu'**au moins une des plaques d'ostéosynthèse (2g, 2h) présente à son extrémité tournée vers l'axe d'articulation (10) un allongement (11) aminci qui présente au moins un trou (3b) pour une vis d'ostéosynthèse (12) et est réalisé sous forme flexible autour de l'os (1).

22. Le kit d'ostéosynthèse selon la revendication 21, **caractérisé en ce que** l'allongement (11) comprend plusieurs trous (3b), et l'allongement (11) est plus mince entre les zones avec les trous (3b) que dans la zone des trous (3b), de telle manière que l'allongement (11) est réalisé sous forme flexible aussi dans le plan de la plaque pour l'adaptation à l'os.

23. Le kit d'ostéosynthèse selon une des revendications précédentes, lequel est pourvu de vis d'ostéosynthèse (12), **caractérisé en ce que** les au moins deux plaques d'ostéosynthèse (2g, 2h) ont une prolongation oblongue le long de respectivement un axe longitudinal (9); et **en ce qu'**à l'état implanté les au moins deux plaques d'ostéosynthèse (2g, 2h) sont agencées dans respectivement un plan au moins approximativement perpendiculaire à l'autre.
